(19) Europäisches Patentamt
European Patent Office
Office européen des brevets

(11) Publication number: 0 095 907 B1

(12) EUROPEAN PATENT SPECIFICATION

(45) Date of publication of patent specification: 27.04.88

(51) Int. Cl.[4]: C 07 D 275/02, A 61 K 31/41, A 61 K 7/48

(21) Application number: 83303064.6

(22) Date of filing: 27.05.83

(54) **Biocidal or biostatic compositions containing 3-isothiazolones, their method of preparation and their uses.**

(30) Priority: 01.06.82 US 383858

(43) Date of publication of application:
07.12.83 Bulletin 83/49

(45) Publication of the grant of the patent:
27.04.88 Bulletin 88/17

(84) Designated Contracting States:
AT BE CH DE FR GB IT LI LU NL SE

(56) References cited:
FR-A-1 555 416
FR-A-2 139 421
US-A-3 870 795
US-A-4 062 946

SEIFEN-ÖLE-FETTE-WACHSE. Kosmetika Aerosole Riechstoffe, vol. 109, no. 7, April 21, 1983, AUGSBURG, (DE), p. 187-190, M. SELTER: "Isothiazolon als =neues= kosmetisches Konservierungsmittel"

(73) Proprietor: Rohm and Haas Company
Independence Mall West
Philadelphia, Pennsylvania 19105 (US)

(72) Inventor: Bayer, Horst Otto
186 Red Rose Drive
Levittown, PA. 19056 (US)
Inventor: Lange, Barry Clifford
19 Canoebirch Road
Levittown, PA. 19057 (US)
Inventor: Petigara, Ramesh Balubhai
2185 Stewart Drive
Hatfield, PA. 19440 (US)

(74) Representative: Angell, David Whilton et al
ROHM AND HAAS (UK) LTD. European
Operations Patent Department Lennig House
2 Mason's Avenue
Croydon CR9 3NB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 907 B1

**Description**

This invention is concerned with biocidal or biostastic compositions containing 3-isothiazolones, their method of preparation and their uses, for example, in the production of cosmetic (including toiletry) and drug (i.e. pharmaceutical) preparations.

The 3-isothiazolones concerned in this invention are known and are of Formula I below:

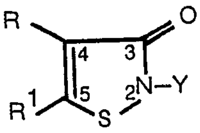

(I)

wherein R and $R^1$ are the same or different and represent hydrogen, halogen (preferably chlorine) or $(C_1—C_4)$alkyl and Y is $(C_1—C_8)$alkyl, $(C_3—C_6)$-cycloalkyl, aralkyl of up to 8 carbon atoms (e.g. benzyl or phenylethyl) or optionally substituted phenyl. The optional substituent(s) may be one or more halogen, cyano, nitro, $(C_1—C_4)$alkyl, $(C_1—C_4)$alkoxy, $(C_1—C_4)$alkylacylamino, $(C_1—C_4)$carbalkoxy or sulfamyl.

The compounds of Formula 1 exhibit biocidal or biostatic activity towards many pest organisms of both animal and vegetable origin such as fungi (e.g mildew), bacteria, algae, slime, and molluscs (e.g. barnacles).

When the 3-isothiazolone of Formula I is one in which Y is $(C_1—C_4)$alkyl, and at least one of R and $R^1$ is halogen (with the other, usually R, hydrogen), the compounds are useful industrial biocides having almost unlimited solubility in water (see U.S. Patent 4,105,431). The less water-soluble, higher alkyl-substituted 3-isothiazolones are generally useful as mildewcides and fungicides in organic solutions and polymer emulsion products such as paints. The higher alkyl-substituted isothiazolones are soluble in various organic solvents such as ethanol, isopropanol and acetone and such solutions may be easily extended with water. Isothiazolones can also be used in solid formulations, preferably absorbed or on in a particulate carrier.

Unfortunately, solutions of the 3-isothiazolones, especially aqueous solutions or solutions in polar organic solvents such as alcohols, are unstable, leading to reduced biological effectiveness. This is especially true of the compounds of Formula I where Y is $(C_1—C_4)$alkyl or $(C_3—C_6)$cycloalkyl. The instability results from an opening of the isothiazolone ring to form linear compounds which do not have the same biological properties as the ring compounds. To inhibit ring cleavage, nitrate salts (such as those of metals such as calcium, copper, magnesium, manganese, nickel, zinc sodium, potassium, iron, barium, cobalt or mixtures of these) can be added to isothiazolone solutions. Thus it is commercially desirable today to formulate many of the 3-isothiazolone biocides in solutions containing water or polar organic solvent or mixtures thereof together with nitrate stabilizers to prevent decomposition of the 3-isothiazolone (see U.S. 3,870,795).

The process used for manufacturing 3-isothiazolones involves amidation of a disulfide followed by the halogenation cyclization of the diamide product as shown below:

*Amidation*

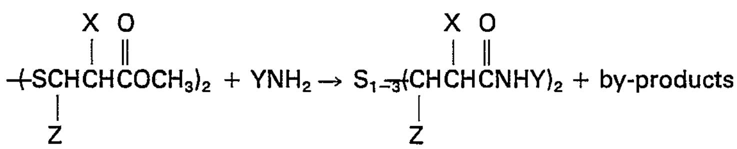

(A)

(disulfide) (amine) (mono)di,tri)sulfidediamide)

*Cyclization*

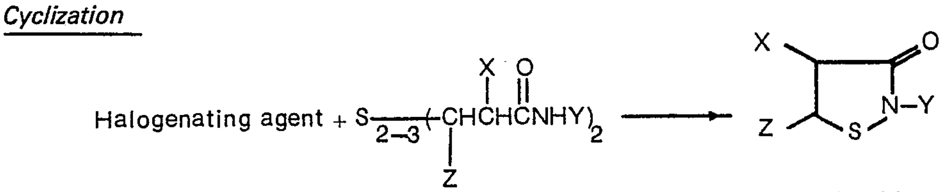


(B)

(3—isothiazolone)

wherein X and Z (R and R′ in the general formula, except for halogen later attached during cyclization) are hydrogen or $(C_1—C_4)$alkyl and Y is as defined in Formula I.

Cyclization is accomplished by contacting the diamide with a halogenating agent. Typical halogenating agents include chlorine, bromine, sulfuryl chloride, sulfuryl bromide, N-chlorosuccinimide, N-bromosuccinimide, and the like. Chlorine and sulfuryl chloride are the preferred halogenating agents. The

amidation of the disulfide intermediate (hereinafter "disulfide") produces the mono(di,tri)sulfidediamide intermediates (hereinafter "diamide") and ultimately the 3-isothiazolone compounds. When prepared according to the above reactions (A) and (B), the 3-isothiazolone component (i.e. the active ingredient which is herein abbreviated as "AI") is generally a mixture comprising two or more different 3-isothiazolone species of Formula I together with various by-products, including some amines which have not heretofore been characterized.

We have now discovered that certain 3-isothiazolone biocides produced using the prior art disulfide intermediate may contain by-product impurities having a secondary or tertiary amine group which, upon exposure to nitrosating conditions, can be converted to nitrosamines which are generally suspected to be possible carcinogens and therefore undesirable especially in preparations to be used in applications where human or animal contact is anticipated. The nitrosamine problem is exacerbated when 3-isothiazolone are present in solutions, either aquoeus solutions or organic solutions or mixtures thereof, wherein it is necessary to incorporate, as ring stabilizer, a nitrate salt, see, e.g., U.S. Patent 4,067,878, or where another nitrosating agent may be present in the isothiazolone composition. When a metal nitrate salt is present as a stabilizer, any by-product secondary or tertiary amine compound present in the 3-isothiazolone reaction mixture is capable of being nitrosated to a nitrosamine.

There follows a description of the process for preparing 3-isothiazolones which utilizes a disulfide intermediate. In such description and elsewhere in this specification quantities are on a weight by weight basis unless stated otherwise.

In preparing 3-isothiazolone industrial biocides by amidation of the usual disulfide intermediates (e.g., dimethyl-3,3-dithiodipropionate), there are found typical levels of nitrosamine precursors between about 0.5% (5,000 ppm) and about 1.1% (11,000 ppm) by weight in the diamide product. After the diamide product is cyclized with a halogenating agent, filtered, neutralized, dissolved in water together with a metal nitrate stabilizer and heat-treated to remove by-product impurities, the final product contains about 6%–16% (by weight of the original precursor) of a nitrosamine. For example, in the case of an original precursor content of 5,000 ppm, the final product (see Experiment 1 below) has been found to contain typically 750 ppm of nitrosamine. Because of the high dilution factor in industrial applications, under use conditions the nitrosamine is rarely present in concentrations greater than parts per billion.

Among the most effective biocides for inhibiting bacterial growth are those containing one or both of the 3-isothiazolones of Formula 1 wherein R is hydrogen, R' is hydrogen or chlorine and Y is methyl, especially a 3-isothiazolone mixture comprised mainly of 5-chloro-2-methyl-4-isothiazolin-3-one and 2-methyl-4-isothiazolin-3-one (mixture dependent upon chlorination (cyclization) conditions), more especially wherein the chlorinated species is between 60% and 90% by weight of the total active ingredients (AI). The process of manufacture includes the following:

*Amidation*

$$-(SCH_2CH_2\overset{O}{\overset{\|}{C}}OCH_3)_2 + 2\,CH_3NH_2 \xrightarrow{\text{(toluene)}}$$

(disulfide)

$$S_n-(CH_2CH_2\overset{O}{\overset{\|}{C}}NHCH_3)_2 + 2CH_3OH + \text{by-products } (\cong 5\%) \quad (1)$$

(diamide) (n = 1, 2, 3)

*Chlorination (Cyclization)*

$$S_{2-3}(CH_2CH_2\overset{O}{\overset{\|}{C}}NHCH_3)_2 + Cl_2\ (3\text{—}6 \text{ moles}) \xrightarrow{\text{(solvent)}}$$

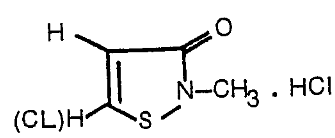

(isothiazolone hydrochloride)

\+ 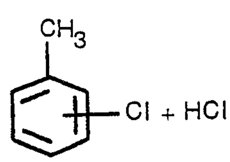 (2)

(when the solvent is toluene)

*Neutralization*

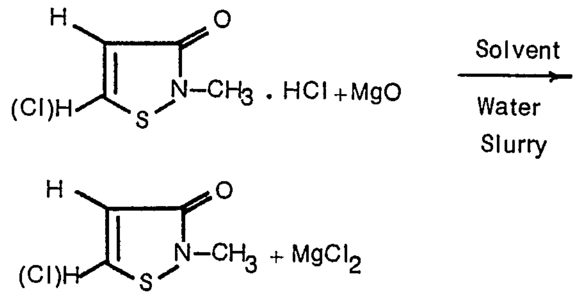


(3)

3—isothiazolone
(technical grade)

The first reaction above (1) produces a mixture containing about 95% mono-, di- and tri-thiodiamides and methanol. Upon cleavage of the disulfide (during amidation), N-methylacrylamide by-product is believed to be formed. Conjugate addition of monomethylamine to this cleavage by-product may lead to the formation of the nitrosamine precursor, N-methyl-3-(N'-methylamino)propionamide, by the following probable reaction:

$$CH_3NH_2 + CH_3NHCOCH{=}CH_2 \rightarrow CH_3NHCH_2CH_2CONHCH_3 \qquad (4)$$

(nitrosamine precursor, MMAP)

N-methylacrylamide will also theoretically add to MMAP produced by reaction (4) above according to the following:

$$CH_3NHCH_2CH_2\overset{\overset{O}{\|}}{C}NHCH_3 + CH_3NHCOCH{=}CH_2 \rightarrow \qquad (5)$$

$$CH_3NHCOCH_2CH_2\overset{\overset{CH_3}{|}}{N}CH_2CH_2CONHCH_3$$

(nitrosamine precursor)

We have now surprisingly identified both of the above nitrosamine precursors, particularly MMAP as being present in the intermediate amide produced when amidating a disulfide starting material. The nitrosamine precursors remain with the AI through chlorination, neutralization and formulation of the 3-isothiazolone composition until the metal nitrate salt is added, at which time nitrosation takes place, principally during heat treatment, (or alternatively during storage) to form a nitrosamine, e.g.:

$$CH_3NHCH_2CH_2CONHCH_3 + NO{\cdot}X \xrightarrow{pH\ 2\text{—}3} CH_3\overset{\overset{NO}{|}}{N}CH_2CH_2CONHCH_3 \qquad (6)$$

The amidation reaction (1) is conducted in an organic solvent, either aliphatic or aromatic or mixtures thereof. Illustrative of the solvents used are methanol, toluene and Laktane. Laktane is a commercial (Exxon) hydrocarbon solvent with a flash point of 25°F, a b.p. range of 102—108°C, and having the composition:

| | |
|---|---|
| paraffins | 28% w/w |
| cycloparaffins | 54% w/w |
| toluene | 18% w/w |

The diamide reaction mixture resulting from amidation has a high solids content. Chlorination of the filtered diamide mixture to form the cyclic 3-isothiazolone hydrochloride mixture (2) is conducted with the diamide in a concentrated slurry in an organic solvent typically toluene, perchloroethylene, ethyl or tributyl

acetate, the reaction preferably involving the concurrent feeding of the diamide slurry and chlorine gas in the proper molar ratio (3—6, preferably 5, mols $Cl_2$/1 mol of amide) to a reactor.

An aqueous slurry of magnesium oxide may be used to neutralize the filtered 3-isothiazolone hydrochloride reaction mixture to form the technical grade product. A metal nitrate stabilizer compound is subsequently added to the technical grade product prior to a final heat treatment step. Heat treatment is effective for removing or decomposing by-products.

It has now been discovered that nitrosamines can be substantially eliminated from 3-isothiazolone products by (I) removing nitrosamine precursor(s) from the diamide intermediate reaction mixture or by (II) inhibiting formation of nitrosamine precursor(s) during the amidation reaction. Alternative processes have been developed for each of (I) and (II).

Accordingly the invention provides a biocidal, or biostatic composition containing (1) 3-isothiazolone, (2) a medium for the 3-isothiazolone, which medium comprises water or polar organic solvent or a mixture thereof, and (3) a ring stabilising amount of a nitrate salt which stabilises the 3-isothiazolone against ring cleavage, said 3-isothiazolone being of Formula I below:

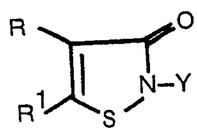

(I)

wherein R and $R^1$ are the same or different and represent hydrogen, halogen or $(C_1—C_4)$alkyl (preferably R is hydrogen and $R^1$ is hydrogen or halogen) and Y is $(C_1—C_8)$ alkyl, (preferably methyl, ethyl, propyl or butyl), $(C_3—C_6)$ cycloalkyl, aralkyl of up to 8 carbon atoms or optionally substituted phenyl groups wherein the optional substituents are as previously defined, and has been prepared by cyclisation with an halogenating agent, of an amide intermediate, characterised in that the content, in the composition, of nitrosamine of the formula

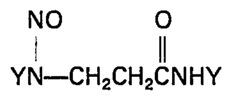

plus precursor(s) thereof is less than 100 parts (on a weight basis) per 150,000 parts of 3-isothiazolone(s) of Formula I.

This level of nitrosamine and nitrosamine precursors is obtainable by the process described below.

Stabilized 3-isothiazolone compositions, as sold for dilution, usually contain substantially 15% AI. Preferred compositions contain less than 50 ppm (and even more preferably less than 20 ppm) of precursors and nitrosamines per 150,000 parts of 3-isothiazolone. As will be demonstrated hereinafter, it is even possible to produce compositions with no detectable nitrosamine or precursor compounds.

Removal or partial removal of the precursor(s) from the diamide intermediate may be accomplished by separation techniques such as (a) ion exchange, (b) crystallization or recrystallization, or (c) solvent extraction (filtration and washing). These techniques are useful with the diamide reaction mixture produced from a disulfide intermediate even when this is carried out in the presence of a nucleophilic scavenger as disclosed later or when utilizing a mercaptan as intermediate as disclosed later for inhibiting formation of the nitrosamine precursor. When the 3-isothiazolone product must be essentially nitrosamine-free, it may be preferred to use a combination of the process using a mercaptan and one or more of the above precursor removal techniques.

Recrystallization of N,N'-dimethyl-3,3'-dithiodipropionamide, from 2-propanol effectively removes the nitrosamine precursor N-methyl-3-(N'-methylamino) propionamide from the reaction mixture of the disulfide and methylamine (see Example 1 below).

Filtration and methanol washing of the N,N'-dimethyl-3,3'-dithiodipropionamide wetcake (see Example 3), below) reduces the precursor N-methyl-3-(N'-methylamino) propionamide level from 5000 ppm to 400 ppm.

Removal from the diamide of the nitrosamine precursor by selective ion exchange of the reaction mixture is also effective (see Examples 4 and 5 below). Treatment of a methanolic solution of N,N'-dimethyl-3,3'-dithiodipropionamide with a sulfonic acid cation exchange resin (Amberlyst 15, Amberlyst being a registered trademark of Rohm and Haas Company) provides god removal of N-methyl-3-(N'-methylamino)propionamide from the reaction mixture of the disulfide with monomethyl amine (see reaction 1, above). The resin may be regenerated with methanolic aqueous hydrogen chloride. The ion exchange process may be represented as follows:

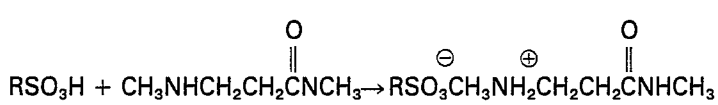

(Resin) (precursor)

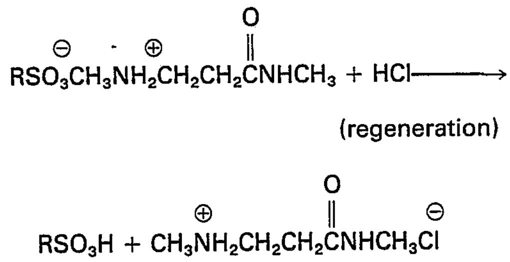


The final product 3-isothiazolone made from the ion exchange-treated intermediate has a much reduced nitrosamine content.

Formation of the nitrosamine precursors can be inhibited by use of a nucleophilic scavenger during the amidation reaction or by selection of different intermediates for the amidation reaction. Useful nucleophilic scavengers are materials which are generally (a) more active than an amine in a Michael addition reaction but (b) which do not degrade the reactants or reaction product of the amidation (see Example 6 below). Most aliphatic and aromatic mercaptans are useful scavengers in relation to the addition reaction to N-alkylacrylamide intermediates, which are the reactive compounds responsible for producing the principal nitrosamine precursors (see reactions 4 and 5 above). Other Michael addition reactants, such as sodium or potassium salts of alcohols are generally not as desirable because of their high reactivity with other starting materials. A higher concentration of the nucleophilic scavenger in the amidation reaction leads to proportionally greater reduction of the nitrosamine precursors:

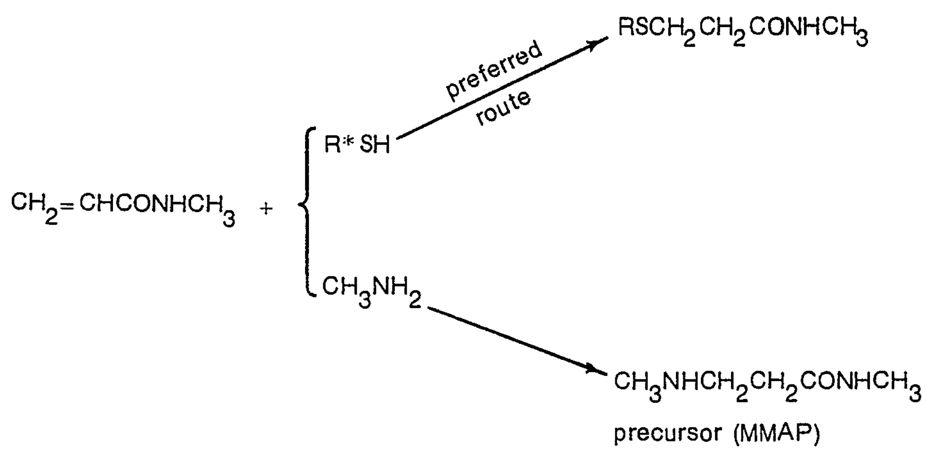


The inherent avoidance of nitrosamine precursor when a mercaptan intermediate [generally of the formula

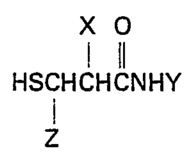

where X and Z are hydrogen or ($C_1$—$C_4$)alkyl and Y is as defined above] is selected in place of the normal disulfide intermediate [which is generally of the formula

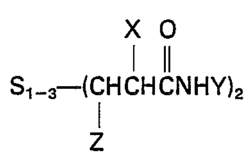

where X and Z are hydrogen or ($C_1$—$C_4$)alkyl and Z is as defined above] in the amidation reaction is an important and unexpected finding of the present invention. A preferred mercaptan intermediate has the formula:

---

R* = e.g., methyl, ethyl, propyl, phenyl, and also —$CH_2CH_2COA$-(alkyl) where A=O or NH.

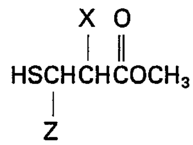

wherein X and Z have the meaning set forth above. The intermediate is essentially half of the conventional disulfide reactant (see reaction A above) used in the amidation reaction but, surprisingly, yields a product much lower in nitrosamine precursor, presumably by the same mechanism as postulated above for the nucleophilic scavengers. In the case of the isothiazolone biocide mixture illustrated above (in reaction 1—3) the mercaptan which may be used has the formula $HSCH_2CH_2COOCH_3$ and is referred to as "MMP" (methyl-3-mercaptopropionate). The remarkable reduction in nitrosamine precursor in the amide when using MMP in place of the usual disulfide is illustrated in the following table:

TABLE I

Precursor* ($CH_3NHCH_2CH_2CONHCH_3$) Level in Intermediate Compared to MMP Process Intermediate

Amidated Disulfide Intermediate
(Reaction 1 above)

| Intermediate Batch | Precursor in Amide Reaction Mixture (ppm) |
|---|---|
| 1 | 5,600 |
| 2 | 4,800 |
| 3 | 6,400 |
| 4 | 4,500 |
| 5 | 5,000 |
| 6 | 10,100 |
| 7 | 11,400 |
| 8 | 5,700 |
| 9 | 9,400 |
| 10 | 6,700 |

Amidated MMP Intermediate
($HSCH_2CH_2CONHCH_3$)

| Intermediate Batch | Precursor in Amide Reaction Mixture (ppm) |
|---|---|
| 1, 2, 3 | 21, 24, 31 |
| 4 | 43 |
| 5 | 37 |
| 6 | 80 |
| 7 | 60 |
| 8 | 70 |
| 9 | <30 |
| 10 | <30 |
| 11 | 110 |

* Precursor = MMAP (see reaction 4, above).

Other advantages of using a mercaptan intermediate (e.g., MMP) may be found in the reaction product of the mercaptan and the amine. In the case of MMP, the amide is a liquid rather than a solid, as is the case with a disulfide intermediate. The difficulties usually encountered in handling, agitating, pumping and reacting a slurry are thus avoided. Further, use of the mercaptan intermediate reduces the amount of halogen (chlorine) needed for cyclization of the amide from the usual 3.0—6.0 mols/mol of intermediate to 2.8—3.4 mols, preferably 3.0 mols, per mol of intermediate.

The nitrosamine precursor by-product of the amidation reaction carries through to the final product, but the dilution of the AI in the product results in a lower concentration. Thus, the ultimate stabilized 3-isothiazolone composition will normally have a nitrosamine concentration of about 15% of the precursor concentration found in the amide. Table II illustrates the different concentrations of the major nitrosamine precursor (MMAP) in the amide compared to the nitrosamine (MMNP) in the corresponding stabilized (nitrate added/heat treated) product for a mixture containing (as the AI) 5-chloro-2-methyl-4-isothiazolin-3-one and 2-methyl-4-isothiazolin-3-one produced from an MMP starting material of the present invention.

TABLE II

Precursor (MMAP) in Amide Intermediate v. Nitrosamine in Stabilized Product

| Amide Intermediate | | | Stabilized 3-Isothiazolone |
|---|---|---|---|
| Sample No. | MMAP (ppm) | | MMNP* (ppm) |
| 1 | 80 | | <3 |
| 2 | <30 | | 2.0 |
| 3 | 60 | | <1.0 |
| 4 | 70 | | 7.3 |
| 5 | <30 | ** | 2.3<br>3.8 |
| 6 | <30 | | 1.9 |
| 7 | 110 | ** | 10.6<br>3.4<br>8.3 |
| 8 | 21 | ** | 1.0<br>1.0 |
| 9 | 24 | | 1.0 |
| 10 | 31 | ** | 1.0<br>2.0 |
| 11 | 31 | | 10.3 |
| 12 | 43 | ** | 11.6<br>7.9<br>3.6 |
| 13 | 37 | ** | 6.0<br>5.3<br>5.6 |

*MMNP = N-methyl-3-(N′-methyl-N′-nitroso)aminopropionamide.
**Intermediate divided for multiple conversions to the final product.

The mechanism for nitrosamine reduction in the above MMP process appears to reside in the reduction of the nitrosamine precursor N-methyl-3-(N′-methyl)aminopropionamide (MMAP) in the amide. The amide disulfide source for the postulated N-methylacrylamide intermediate (reaction 4 above) is reduced to a minor reaction by-product. Also, the MMP starting material and N-methyl-3-mercaptopropionamide appear to compete successfully with monomethylamine to consume N-methylacrylamide and hence avoid formation of MMAP. These postulated alternative routes are as follows:

$$CH_3NHCOCH{=}CH_2 + HSCH_2CH_2CO_2CH_3 \rightarrow CH_3NHCOCH_2CH_2SCH_2CH_2CO_2CH_3$$

$$CH_3NHCOCH=CH_2 + HSCH_2CH_2CONHCH_3 \rightarrow (CH_3NHCOCH_2CH_2)_2S$$

The net result is the reduction of MMAP levels from about 5,000—11,000 ppm for the disulfide process to about <100 ppm in the MMP process.

The heat treatment carried out after the addition of the nitrate stabilizer is preferably performed for a duration and at a temperature and pressure such as will result in the same degree of by-product removal or decomposition as would be achieved by a heat treatment for 4 hours at 95°C and under atmospheric pressure.

The following comparative experiments and examples are offered to illustrate this invention.

Experiment 1
(Comparative experiment using a disulfide intermediate)

Step 1: Amidation Preparation of N,N′-dimethyl-3,3′-dithiodipropionamide Intermediate

Charged to a vapor-tight reaction kettle was dimethyl-3,3′-dithiodipropionate 45.8 kg (101 lb, 0.424 mol), laktane 59.4 kg (131 lb) and methanol 2.3 kg (5.06 lb). The mixture was cooled to 15—20°C with agitation. Monomethylamine 14.9 kg (32.8 lb. 1.06 mol) was added beneath the surface of the reaction mixture with agitation at 15—20°C and 3441—6892 N/m$^2$ (5—10 psi) over 2 hr. After completing the monomethylamine addition, the mixture was stirred at 15—20°C for 10 hr. A thick, pale-yellow slurry was obtained. At this time the unreacted monomethylamine and methanol by-product were distilled from the mixture at ~ 0.133 Bar (100 mmHg). After the distillation period, the yellow slurry was rotary vacuum dried and isolated without washing to provide crude, dry N,N′-dimethyl-3,3′-dithiodipropionamide 45.4 kg (100 lb, 100% yield), containing 5,000 ppm N-methyl-3-(N′-methyl)aminopropionamide.

Step 2: Chlorination (cyclization)

Preparation of a mixture of 5-chloro-2-methyl-4-isothiazolin-3-one hydrochloride and 2-methyl-4-isothiazolin-3-one hydrochloride.

A slurry of the crude N,N′-dimethyl-3,3′-dithiodipropionamide reaction product of Step 1 was diluted with toluene and chlorinated to yield a slurry containing 5-chloro-2-methyl-4-isothiazolin-3-one hydrochloride and 2-methyl-4-isothiazolin-3-one hydrochloride and mother liquor.

Step 3: Filtration and Neutralization

The chlorinated slurry from Step 2 was filtered and neutralized with a magnesium oxide slurry to form the technical grade product.

Step 4: Formulation and Heat Treatment (Stabilization)

The technical grade product made in Step 3 was formulated by adding magnesium nitrate hexahydrate, and transferring the mixture to a heat treatment kettle equipped with an agitator and a reflux condenser. The product was heat treated for 4 hours and then allowed to cool to room temperature. The batch was filtered to remove small amounts of suspended solids. This gave a product with the following AI and nitrosamine analysis:

| Components | |
|---|---|
| 5-chloro-2-methyl-4-isothazolin-3-one | 9.8% by wt |
| 2-methyl-4-isothiazolin-3-one | 6.0% by wt |
| Nitrosamine[a] | 750 ppm |

Experiment 2
(Further comparative experiment using a disulfide intermediate)

Step 1: Amidation

Into a three-liter, 4-necked flask equipped with a mechanical stirrer, thermometer, gas dispersion tube and dry ice condenser with nitrogen inlet adapter, was placed dimethyl-3,3′-dithiodipropionate (1,062.5 g, 4.46 mol), toluene (535.0 g) and methanol (55.0 g). The apparatus was purged with nitrogen and the mixture was cooled to 10°C. Monomethylamine (346.0 g, 11.14 mol) was added through the gas dispersion tube with stirring at 10—20°C over 2 hrs. After completing the monoethylamine addition, the mixture was stirred at 20°C for 20 hrs. to complete the reaction. A thick, pale yellow slurry was obtained. At this time the unreacted monomethylamine and methanol by-product were distilled from the mixture at ~ 0.133 Bar (100 mm Hg). The crude dry N,N′-dimethyl-3,3′-dithiodipropionamide intermediate (1,022.4 g, 97% yield) contained 11,000 ppm N-methyl-3-(N′-methyl)aminopropionamide.

[a] N-methyl-3-(N′-methyl-N′-nitroso)aminopropionamide (MMNP).

A portion of the intermediate slurry was filtered, washed with toluene and dried. The dry intermediate contained 8,000 ppm of N-methyl-3-(N′-methyl)aminopropionate.

Step 2: Chlorination

Preparation of a mixture of 5-chloro-2-methyl-4-isothiazolin-3-one hydrochloride and 2-methyl-4-isothiazolin-3-one hydrochloride.

A one-liter 3-necked round bottom flask was equippped with an overhead agitator, a feed line (outlet) and a condenser with a drying tube. Into this flask, 635.8 g of a slurry of N,N′-dimethyl-3,3′-dithiodipropionamide (with 8,000 ppm precursor) in toluene was placed and agitated.

A one-liter, 5-necked resin kettle (i.e., a chlorinator) was equipped with an agitator, a fritted glass gas dispersion tube for $Cl_2$ inlet, a thermometer, a condenser attached to an off-gas scrubber, and a feed line-inlet for intermediate slurry. The kettle was jacketed for ice-water circulation. The cooling system maintained the chlorination batch at 25—30°C. The chlorinator was charged with a 108 g of toluene as a heel, and the agitator was started.

The slurry and $Cl_2$ were fed concurrently at a molar feed ratio of 5.2. Thus, 453 g of the slurry was charged over a 55-minute period at a rate of about 8.2 g/min., while 227 g of $Cl_2$ (gas) was fed at a rate of about 4.1 g/min., using a calibrated flowmeter.

Step 3: Filtration and Neutralization

To the agitated chlorination slurry 20 g of water was added gradually. After 10 min. of agitation, the batch was allowed to settle, and the mother liquor was siphoned out using a dipstick. An additional 45 g of water was added, and additional mother liquor was removed.

To the hydrochloride wet cake was added 116 g of water. The mixture was neutralized to a pH 4.5 by gradually adding an aqueous MgO slurry. The neutralized material was transferred to a separatory funnel and a 469 g of an aqueous technical grade material was separated from the organic layer:

| Active Ingredient | Wt % |
|---|---|
| 5-chloro-2-methyl-4-isothiazolin-3-one | 17.1 |
| 2-methyl-4-isothiazolin-3-one | 5.5 |

Step 4: Formulation and Heat Treatment (Stabilization)

The pH of the above technical grade material was adjusted to 2.9, and 46.5 g of magnesium nitrate hexahydrate and 7.24 g of water were added to 100 g of the AI with agitation to give a solution with the following composition:

| Component | Nominal Conc., Wt % |
|---|---|
| Total AI | 15.2 |
| $Mg(NO_3)_2$ | 17.4 |

The above formulated product was transferred to a 500 ml 3-necked round bottom flask equipped with an overhead agitator, a water-cooled condenser and a thermometer attached to a thermo-watch and pneumatic pot lifter assembly supporting a heating mantle.

The formulated product was heat-treated at 95°C for 4 hrs. The product, 153.7 g, was filtered to remove any trace amounts of solids, and analyzed.

Analysis:

| Components | |
|---|---|
| 5-chloro-2-methyl-4-isothiazolin-3-one | 10.1% by wt. |
| 2-methyl-4-isothiazolin-3-one | 5.0% by wt. |
| Nitrosamine* | 1200 ppm |

---

NO
|
* $CH_3N—CH_2CH_2CONHCH_3$

Example 1

Recrystallization of Crude N,N'-Dimethyl-3,3'-dithiodipropionamide (Removal of Nitrosamine Precursor)

Crude diamide intermediate (disulfide produced as in Experiment 1 above), 1400 g, was dissolved in 1750 g of boiling 2-propanol and the solution was filtered rapidly through a pre-heated Buchner funnel. The filtrate was kept overnight at 5—10°C in a refrigerator. The crystalline product was collected by filtration on a Buchner funnel, washed with a 560 g portion of cool methanol and dried on a rotary evaporator (35°C/0.026 bar) (20 mm of Hg)/2 hours) to give 995 g of 99.1% pure intermediate m.p. 113—115°C.

Next, 789.2 g of the crystallized diamine intermediate was recrystallized from 2300 g of boiling 2-propanol to give 685.6 g of 99.9$^+$% w/w amide intermediate, m.p. 113—115°C.

| | | | | | |
|---|---|---|---|---|---|
| Anal. calcd. for $C_8H_{16}N_2O_2S_2$: | C, 40.65; | H, 6.82; | N, 11.85; | O, 13.54; | S, 27.13. |
| Found: | C, 40.29; | H, 6.83; | N, 11.61; | O, 13.57; | S, 27.38. |

The N-methyl-3-(N'-methyl)aminopropionamide content was 0 ppm.

(b) Nitrosamine-Free Product, Using Pure Amide Intermediate

Following the chlorination procedure described in Experiment 2, 152.6 g of recrystallized pure diamide was chlorinated; the chlorination slurry was filtered and neutralized to give 537.9 g of technical grade product, with a total Al of 21.5%.

A 350 g portion of this technical grade product was formulated by dissolving 145.5 g magnesium nitrate hexahydrate in the product. The formulated product was heat-treated at 95°C for 4 hours to give 495 g of 3-isothiazolone composition with the following ingredients:

| | |
|---|---|
| 5-chloro-2-methyl-4-isothiazolin-3-one | 10.3% by wt. |
| 2-methyl-4-isothiazolin-3-one | 5.0% by wt. |
| Nitrosasmine ppm | 0 |
| Nitrosamine precursor(s) | none detected. |

Example 2

Nitrosamine-free Products, from Pure Active Ingredient Isothiazolones

Alternatively, pure 2-methyl-4-isothiazolin-3-one, and pure 5-chloro-2-methyl-5-isothiazolin-3-one, can be obtained and formulated as above to give pure product. This material, with or without the 95°C/4 hour heat treatment, is found to be free of nitrosamines and nitrosamine precursors.

Example 3

(Precursor Removal by Solvent Extraction)

Into a three-liter, 4-necked flask equipped with a mechanical stirrer, thermometer, gas dispersion tube and dry ice condenser with nitrogen inlet adapter, was placed dimethyl-3,3'-dithiodipropionate (1062.5 g, 4.45 mol) toluene (295.0 g) and methanol (295.0 g). The apparatus was purged with nitrogen and the mixture was cooled to 10°C. Monomethylamine (304.7 g, 9.81 mol) was added through the gas dispersion tube with stirring at 10—20°C over 2 hrs. After completing the monomethylamine addition, the mixture was stirred at 20°C for 20 hrs. to complete the reaction. A thick, pale yellow slurry was obtained. At this time, the unreacted monomethylamine, methanol and some toluene were distilled from the mixture at 0.133 Bar (100 mmHg) over 8 hrs. The resulting slurry was rotary evaporated to give crude N,N'-dimethyl-3,3'-dithiodipropionamide (1058.9 g, 100% yield), containing 5,000 ppm N-methyl-3-(N'-methyl)amino-propionamide. A portion of the crude product (353.0 g was slurried in 784.5 g toluene) and vacuum filtered through a 2,000 ml course sintered glass funnel to give a toluene wet cake (419.1 g). The toluene wet cake was washed with cold (0°C) methanol (352.4 g) to give the methanol wet cake (315.2 g). The methanol wet cake was dried in a vacuum desiccator at ambient temperature to give the washed, dried amide intermediate (251.1 g), 71% recovery), containing 400 ppm N-methyl-3-(N'-methyl)aminopropionamide.

Following the process steps 2, 3 and 4 described in Experiment 2 above, the dry N,N'-dimethyl-3,3'-dithiodipropionamide was converted to give 260 g of 3-isothiazolone product (pH 2.1) with the following composition:

| Components | |
|---|---|
| 5-chloro-2-methyl-4-isothiazolin-3-one | 12.0% by wt. |
| 2-methyl-4-isothiazolin-3-one | 2.7% by wt. |
| Nitrosamine | 25 ppm |
| Nitrosamine precursor(s) | none detected |

Example 4

(Precursor Removal by Ion Exchange)

Unfiltered Laktane (Experiment 1 above) process N,N'-dimethyl-3,3'-dithiodipropionamide was rotary evaporated in the laboratory to constant weight. The dried intermediate contained 10,000 ppm N-methyl-3-(N'-methyl)aminopropionamide.

Conditioned Amberlyst 15 sulfonic acid ion exchange resin (22.2 g of 45.1% w/w material in water, 10.0

g of dry resin) was washed into a 50 ml burette (1 cm diameter) with methanol (25 ml). The resin was rinsed on the column with methanol (500 ml) to give a resin bed volume of 28 ml.

The dry intermediate was dissolved in methanol to give a 19.9% w/w solution. This solution was passed through the resin column at ambient temperature and atmosphereic pressure at a flow rate of 9.21 bed volumes per minute. The resin became saturated with N-methyl-3-(N′-methyl)aminopropionamide and break-through occurred after collecting 34 bed volumes. The total quantity of methanolic solution treated up to the break-through point was 737.2 g. Rotary evaporation of the eluent allowed recovery of the crude N,N′-dimethyl-3,3′-dithiodipropionamide (146.5 g, 99.9% recovery), containing 380 ppm N-methyl-3-(N′-methyl)aminopropionamide. Thus crude product, when converted to a nitrate-stabilized 3-isothiazolone composition by the procedure of Example 1(b) results in a composition in accordance with the invention.

Example 5

(Precursor Removal by Ion Exchange)

Into a one-liter, 4-necked flask equipped with a mechanical stirrer, thermometer, gas dispersion tube and dry ice condenser with nitrogen inlet adapter, was placed dimethyl-3,3′-dithiodipropionate (215.5 g, 0.904 mol) and methanol (118.0 g). The apparatus was purged with nitrogen and the mixture was cooled to ~10°C. Monomethylamine (70.0 g, 2.25 mol) was added through the gas dispersion tube with stirring at 10—20°C over 2 hrs. After completing the addition, the mixture was stirred at 20°C for 20 hrs, to complete the reaction. A thick, pale yellow slurry was obtained. At this time the unreacted monomethylamine and some methanol were distilled from the mixture at ~0.133 Bar (100 mmHg). After distillation, a portion of the slurry (17.2 g) was rotary evaporated to constant weight (8.0 g), giving the concentration of crude product in the slurry at 47% w/w. The 47% w/w slurry was 436.0 g, corresponding to 204.9 g of crude N,N′-dimethyl-3,3-dithiodipropionamide (96% yield). The dry, crude product contained 9,000 ppm N-methyl-3-(N′-methyl)aminopropionamide.

Conditioned Amberlyst 15 sulfonic acid ion exchange resin (22.2 g of 45.1% w/w material in water, 10.0 g of dry resin) was washed into a 50 ml buret (1 cm diameter) with methanol (25 ml). The resin was rinsed on the column with methanol (500 ml) to give a resin bed volume of 28 ml.

The 47% w/w slurry in methanol was further diluted with methanol to provide a 20.0% w/w solution. This solution was passed through the resin column at ambient temperature and atmospheric pressure at a flow rate of 0.21 bed volumes per minute. The resin became saturated with N-methyl-3-(N′-methyl)amino-propionamide and break-through occurred after collecting 18 bed volumes of eluent. The total quantity of methanolic N,N′-dimethyl-3,3′-dithiodipropionamide solution treated up to the break-through point was 315.0 g (63.0 of AI). Rotary evaporation of the eluent allowed recovery of the crude N,N-methyl-3,3′-dithio-propionamide (63.0 g; 100% recovery), containing 300 ppm N-methyl-3-(N′-methyl)aminopropionamide.

Following the procedure described in Example 2, 73.3 g of the above-mentioned intermediate was chlorinated, filtered and neutralized to give 217.5 g of Tech grade product.

A 60 g sample of this Tech grade product was formulated with 29.4 g of $Mg(NO_3)_2 \cdot 6H_2O$ and 7.8 g of water. The formulated product was heat-treated at 95°C for 4 hours, cooled and filtered to give about 97 g of 3-isothiazolone with the following composition:

| | |
|---|---|
| 5-chloro-2-methyl-4-isothiazolin-3-one | 11.7% by wt. |
| 2-methyl-4-isothiazolin-3-one | 3.5% by wt. |
| Nitrosamine, ppm | 33 |
| Nitrosamine precursor(s) | none detected. |

Example 6

(Inhibition of Precursor by Nucleophilic Scavenger)

Following the procedure of Experiment 2, Step 1, dimethyl-3,3′-dithiodipropionate (212.5 g, 0.892 mol), methyl-3-mercaptopropionate (18.4 g, 0.153 mol) and monomethylamine (69.0 g, 2.22 mol) were reacted. The intermediate slurry was rotary evaporated to give crude N,N′-dimethyl-3,3′-dithiodipropionamide (224.1 g, 98% crude yield), containing 1,000 ppm N-methyl-3-(N′-methyl)aminopropionamide. When the above steps were repeated using higher levels of a nucleophilic mercapto scavenger, the results established an essentially proportional reduction of nitrosamine precursor as higher concentrations of nucleophilic scavenger were used. In this manner, there is produced an essentially nitrosamine-free and nitrosamine precursor-free 3-isothiazolone composition in accordance with the invention.

Example 7

(Inhibition of Precursor by Mercaptan Reactant Route)

Step 1: Preparation of N-methyl-3-mercaptopropionamide (MMPA)

Into a one-liter, 4-necked flask equipped with a mechanical stirrer, thermometer, gas dispersion tube, and dry ice condenser with nitrogen inlet adapter, was placed methyl-3-mercaptopropionate (MMP, 504.7 g, 4.20 mol). The vessel was purged with nitrogen and the liquid was cooled to 10°C. Monomethylamine (163.0 g, 5.25 mol) was added through the gas dispersion tube with stirring at 10—20°C over 1 hr. After completing the addition, the mixture was stirred at 20°C for 20 hrs to complete the reaction. At this time the

methanol by-product and unreacted monomethylamine were distilled from the mixture at ~0.133 Bar (100 mmHg). The resulting mixture was rotary evaporated to give crude N-methyl-3-mercaptopropionamide (500.8 g, 100% yield), containing <30 ppm N-methyl-3-(N'-methyl)aminopropionamide.

Step 2: Chlorination

The procedure described in Experiment 2 was modified in that N-methyl-3-mercaptopropionamide (MMPA) was used in place of N,N'-dimethyl-3,3'-dithiodipropionamide. Thus, a 31% solution of N-methyl-3-mercaptopropionamide in toluene and $Cl_2$ were fed concurrently at a molar feed ratio of about 3.2.

To 59.5 g of a toluene heel, 398.9 g of 31% MMPA solution was charged over a period of 55 minutes at a rate of 7.1 g per minute, while a 220 g of $Cl_2$ was fed concurrently at a feed rate of 4.0 g per minute.

Step 3: Filtration and Neutralization

Following the procedure described in Experiment 2, the chlorination slurry made above was worked-up and neutralized to give 355 g of Technical grade 3-isothiazolone with the following composition:

| | |
|---|---|
| 5-chloro-2-methyl-4-isothiazolin-3-one | 16.0% by wt. |
| 2-methyl-4-isothiazolin-3-one | 5.3% by wt. |

Step 4: Formulation and Heat Treatment (Stabilization)

Following the procedure described in Experiment 2, a 200 g portion of the Tech grade material made above was formulated by adding 87.7 g $Mg(NO_3)_2 \cdot 6H_2O$ and 2.1 g water. The formulated product was heat-treated, cooled and filtered to give 289 g of 3-isothiazolone product with the following composition:

| | |
|---|---|
| 5-chloro-2-methyl-4-isothiazolin-3-one | 12.2% by wt. |
| 2-methyl-4-isothiazolin-3-one | 3.9% by wt. |
| Nitrosamine | 3.9 ppm |
| Nitrosamine precursor(s) | none detected. |

Example 8

(Preparation of N-(n-octyl)-3-mercaptopropionamide by Mercaptan Reactant Route)

In a small multi-necked reaction vessel equipped with a magnetic stirrer and gas inlet was placed isopropanol (2 ml), methyl-3-mercaptopropionate (2.0 g, 16.64 mmol), and n-octylamine (2.19 g, 16.94 mmol). The reaction vessel was connected to a trap containing bleach to trap mercaptan vapors, and the reaction was stirred for 19.5 hours while the reaction temperature was held at 30—35°C. Methylene dichloride was added and the crude product was transferred to a round bottom flask. Evaporation of the solvent under reduced pressure yielded crude N-(n-octyl)-3-mercaptopropionamide as an oily white solid in essentially quantitative yield.

The N-(n-octyl)-3-mercaptopropionamide thus prepared is converted to a composition containing the corresponding 2-n-octyl-isothiazolin-3-one substantially free of nitrosamine and nitrosamine precursor, i.e. a composition in accordance with the invention.

Example 9

(Preparation of N-propyl-3-mercaptopropionamide by Mercaptan Reaction Route)

In a small multi-necked reaction vessel equipped with a magnetic stirrer, reflux condenser, and gas-inlet was placed isopropanol (2 ml), n-propylamine (1.00 g, 16.92 mmol), and methyl-3-mercaptopropionate (2.0 g, 16.64 mmol). The reaction vessel was connected to a trap containing bleach, and the reaction was stirred at 30—35°C for 19.5 hours. The crude reaction mixture was concentrated under reduced pressure to remove excess amine, solvent and methanol. A light yellow liquid was obtained which was essentially all N-propyl-3-mercaptopropionamide. The N-propyl-3-mercaptopropionamide thus prepared is converted to a composition containing the corresponding N-propylisothazolin-3-one substantially free of nitrosamine and nitrosamine precursor, i.e. a composition in accordance with the invention.

The word "Laktane" is a trademark which may be registered in one or more of the designated states.

**Claims**

1. A process for preparing a 3-isothiazolone composition containing (a) 3-isothiazolone of the formula:

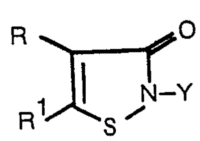

(I)

wherein R and $R^1$ are the same or different and represent hydrogen, halogen or $(C_1—C_4)$alkyl and Y is $(C_1—C_8)$alkyl, $(C_3—C_6)$cycloalkyl, aralkyl of up to 8 carbon atoms or phenyl, optionally substituted with one

or more halogen, cyano, nitro, ($C_1$—$C_4$)alkyl, ($C_1$—$C_4$)alkoxy, ($C_1$—$C_4$)alkylacylamino, ($C_1$—$C_4$)carbalkoxy or sulfamyl, (b) a medium for the isothiazolone, which medium comprises water, polar organic solvent or a mixture thereof and (c) an appropriate amount of nitrate salt as a stabilizer for the isothiazolone, wherein the isothiazolone is prepared by cyclisation, with an halogenating agent, of an intermediate product containing an amide, characterised in that a content of nitrosamine by-product impurity plus precursor(s) thereof in the final stabilised product below 100 parts (on a weight basis) per 150,000 parts of the 3-isothiazolone is achieved (A) by using as the amide intermediate a diamide of the formula

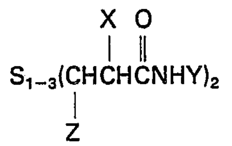

where X and Z are hydrogen or ($C_1$—$C_4$)alkyl and Y is as defined above, and, immediately prior to cyclisation, removing, wholly or in part, precursor(s) of the nitrosamine from the diamide intermediate by (i) ion exchange, (ii) recrystallization and/or (iii) solvent extraction or (B) by avoiding or reducing formation of such precursor(s) during the amidation reaction by which the amide intermediate is formed by: (a)(i) preparing the 3-isothiazolone from an intermediate product containing a diamide as defined in (A) above and (ii) forming the intermediate product by the reaction of an appropriate disulfide and amine in the presence of a nucleophilic scavenger which is a material, such as aliphatic or aromatic mercaptan, which is generally more active than an amine in a Michael addition reaction but which does not degrade the reactants or product of the reaction; or by (b) cyclization, with an halogenating agent, of a mercapto-amide of the formula HSC(Z)HC(X)HC(O)NHY where X, Y and Z are as defined above.

2. A process according to claim 1 wherein the nitrosamine impurity comprises nitrosamine of the general formula

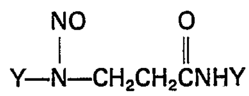

and/or the precursor(s) comprise at least one compound of the formula

$Y—NHCH_2CH_2CONHY$ or 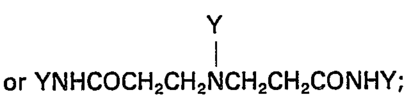;

where Y is as defined in claim 1.

3. A process according to claim 1 and 2 wherein the 3-isothiazolone comprises a mixture of 5-chloro-2-methyl-4-isothiazolone and 2-methyl-4-isothiazilin-3-one and Y in the formulae in claim 2, is —$CH_3$.

4. A process according to claim 1(B)(a) or 1(B)(b) wherein (a) ion exchange, (b) recrystallization and/or (c) solvent extraction is/are also used to effect partial or complete removal of nitrosamine precursor(s) prior to cyclisation.

5. A process according to any preceding claim wherein the content of nitrosamine plus precursor(s) is less than 50 parts, preferably less than 20 parts, per 150,000 parts of the 3-isothiazolone (by weight).

6. A biocidal or biostatic composition containing (1) 3-isothiazolone of the formula

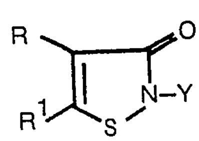

(I)

wherein R and $R^1$ are the same or different and represent hydrogen, halogen or ($C_1$—$C_4$)alkyl and Y is ($C_1$—$C_8$)alkyl, ($C_3$—$C_6$)cycloalkyl, aralkyl of up to 8 carbon atoms or phenyl optionally substituted with one or more halogen, cyano, nitro, ($C_1$—$C_4$)alkyl, ($C_1$—$C_4$)alkoxy, ($C_1$—$C_4$)alkylacylamino, ($C_1$—$C_4$)carbalkoxy or sulfamyl, (2) a medium for the isothiazolone, which medium comprises water or polar organic solvent or a mixture thereof, (3) a ring-stabilizing amount of a nitrate salt which stabilizes the 3-isothiazolone against ring cleavage wherein the 3-isothiazolone has been prepared by cyclization, with an halogenating agent, of an amide intermediate, characterised in that the content of nitrosamine of the formula

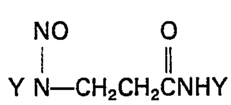

and precursor(s) thereof in the composition is at a level, below 100 parts (on a weight basis) per 150,000 parts of 3-isothiazolone of the above formula, said level being obtainable by (A) using as the amide intermediate a diamide of the formula

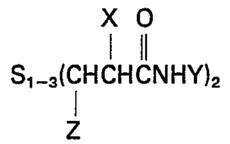

where X and Z are hydrogen or ($C_1$—$C_4$)alkyl and Y is as defined above, and, immediately prior to cyclisation, removing, wholly or in part, precursor(s) of the nitrosamine from the diamide intermediate by (i) ion exchange, (ii) recrystallization and/or (iii) solvent extraction or (B) by avoiding or reducing formation of such precursor(s) during the amidation reaction by which the amide intermediate is formed by: (a)(i) preparing the 3-isothiazolone from an intermediate product containing a diamide as defined in (A) above and (ii) forming the intermediate product by the reaction of an appropriate disulfide and amine in the presence of a nucleophilic scavenger which is a material, such as aliphatic or aromatic mercaptan, which is generally more active than an amine in a Michael addition reaction but which does not degrade the reactants or product of the reaction; or by (b) cyclization, with an halogenating agent, of a mercapto-amide of the formula HSC(Z)HC(X)HC(O)NHY where X, Y and Z are as defined above.

7. A composition according to claim 6 containing one or both of the 3-isothiazolones of the given formula wherein R is hydrogen, $R^1$ is hydrogen or chlorine and Y is methyl.

8. A composition according to claim 7 wherein the 3-isothiazolone comprises a mixture of 5-chloro-2-methyl-4-isothiazolin-3-one and 2-methyl-4-isothiazolin-3-one, the mixture containing 60 to 90% by weight of the chloro compound.

9. A composition according to any of claims 6 to 8 wherein the content of nitrosamine and precursor(s) thereof is less than 50 parts per 150,000 parts of 3-isothiazolone, by weight.

10. A composition according to claim 9 wherein the content of nitrosamine and precursor(s) thereof is less than 20 parts per 150,000 parts of 3-isothiazolone by weight.

11. The use of a composition according to any of claims 6 to 10 in the production of a cosmetic (including toiletry) or pharmaceutical preparation in a biostatic or biocidal amount.

**Patentansprüche**

1. Verfahren zur Herstellung einer 3-Isothiazolonzubereitung, die (a) 3-Isothiazolon der Formel

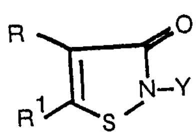

(I)

enthält, worin R und $R^1$ gleich oder verschieden sind und Wasserstoff, Halogen oder ($C_1$—$C_4$)Alkyl bedeuten und Y ($C_1$—$C_8$)Alkyl, ($C_3$—$C_6$)Zykloalkyl, Aralkyl mit bis zu 8 Kohlenstoffatomen oder Phenyl, gegebenenfalls substituiert mit einem oder mehreren der Substituenten Halogen, Cyano, Nitro, ($C_1$—$C_4$)Alkyl, ($C_1$—$C_4$)Alkoxy, ($C_1$—$C_4$)Alkylacylamino, ($C_1$—$C_4$)Carbalkoxy oder Sulfamyl ist, (b) ein Medium für das Isothiazolon, wobei dieses Medium aus Wasser, einem polaren organischen Lösungsmittel oder einer Mischung davon besteht, und (c) eine geeignete Menge eines Nitratsalzes als Stabilisierungsmittel für das Isothiazolon enthält, wobei das Isothiazolon hergestellt wird durch Zyklisierung eines Zwischenprodukts, das ein Amid enthält, mit einem Halogenierungsmittel, dadurch gekennzeichnet, daß ein Gehalt an Nitrosaminnebenproduktverunreinigung plus Vorläufer davon in dem fertigen stabilisierten Produkt von weniger als 100 Teilen (auf Gewichtsbasis) pro 150000 Teile des 3-Isothiazolons erreicht wird (A) durch Verwendung eines Diamids der Formel

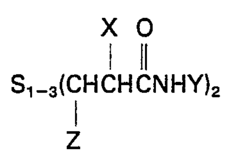

als Amidzwischenprodukt, worin X und Y Wasserstoff oder ($C_1$—$C_4$)Alkyl sind und Y die vorstehend angegebenen Bedeutungen besitzt, und unmittelbar vor der Zyklisierung ganz oder teilweise einer oder mehrere Vorläufer des Nitrosamins aus dem Diamidzwischenprodukt entfernt wird (werden) durch (i) Ionenaustausch, (ii) Umkristallisation und/oder (iii) Lösungsmittelextraktion, oder (B) durch Vermeiden oder verringerte Bildung eines oder mehrerer derartiger Vorläufer während der Amidierunsreaktion, bei

welcher das Amidzwischenprodukt gebildet wird, durch (a) (i) Herstellung des 3-Isothiazolons aus einem Zwischenprodukt, welches das vorstehend unter (A) beschriebene Diamid enthält und (ii) Bildung des Zwischenprodukts durch die Reaktion eines geeigneten Disulfids und eines Amins in Gegenwart eines nukleophilen Spülmittels, das ein Material ist, wie ein aliphatisches oder aromatisches Mercaptan, das im allgemeinen aktiver ist als ein Amin in einer Michael-Additionsreaktion, das jedoch nicht die Reaktanten oder das Produkt der Reaktion zersetzt, oder (b) durch Zyklisierung eines Mercaptoamids der Formel

HSC(Z)HC(X)HC(O)NHY

worin X, Y und Z die vorstehend angegebenen Bedeutungen besitzen, mit einem Halogenierungsmittel.

2. Verfahren nach Anspruch 1, wobei die Nitrosaminverunreinigung aus einem Nitrosamin der allgemeinen Formel

NO O
| ||
Y—N—$CH_2CH_2$CNHY

besteht und/oder der oder die Vorläufer aus wenigstens einer Verbindung der Formeln

Y
|
Y—$NHCH_2CH_2CONHY$ oder $YNHCOCH_2CH_2NCH_2CH_2CONHY$;

worin Y die in Anspruch 1 angegebene Bedeutung besitzt, besteht (bestehen).

3. Verfahren nach Anspruch 1 oder 2, wobei das 3-Isothialon aus einer Mischung aus 5-Chlor-2-methyl-4-isothiazolon und 2-Methyl-4-isothiazolin-3-on besteht und Y in den Formeln im Anspruch 2 für —$CH_3$ steht.

4. Verfahren nach Anspruch 1(B)(a) oder 1(B)(b), worin (a) Ionenaustausch, (b) Umkristallisation und/oder (c) Lösungsmittelextraktion angewendet wird (werden), um die teilweise oder vollständige Entfernung von Nitrosamin-Vorläufer(n) vor der Zyklisierung zu bewirken.

5. Verfahren nach einem der vorhergehenden Ansprüche, wobei der Gehalt an Nitrosamin plus Vorläufer(n) weniger als 50 Teile, vorzugsweise weniger als 20 Teile, pro 150 Teile des 3-Isothiazolons (bezogen auf das Gewicht) ist.

6. Biozides oder biostatisches Mittel, enthaltend (1) 3-Isothiazolon der Formel

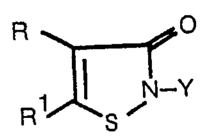

(I)

worin R und $R^1$ gleich oder verschieden sind und für Wasserstoff, Halogen oder ($C_1$—$C_4$)Alkyl stehen und Y ($C_1$—$C_8$)Alkyl, ($C_3$—$C_6$)Zykloalkyl, Aralkyl mit bis zu 8 Kohlenstoffatomen oder Phenyl ist, gegebenenfalls substituiert mit einem oder mehreren der Substituenten Halogen, Cyano, Nitro, ($C_1$—$C_4$)Alkyl, ($C_1$—$C_4$)Alkoxy, ($C_1$—$C_4$)Alkylacylamino, ($C_1$—$C_4$)Carbalkoxy oder Sulfamyl, (2) ein Medium für das Isothiazolon, wobei das Medium aus Wasser oder einem polaren organischen Lösungsmittel oder einer Mischung davon besteht, (3) eine Ring-stabilisierende Menge eines Nitratsalzes, welches das 3-Isothiazolon gegenüber einer Ringspaltung schützt, wobei das 3-Isothiazolon hergestellt worden ist durch Zyklisierung eines Amidzwischenprodukts mit einem Halogenierungsmittel, dadurch gekennzeichnet, daß der Gehalt des Nitrosamins der Formel

NO O
| ||
Y N—$CH_2CH_2$CNHY

und des oder der Vorläufer davon in der Zubereitung sich auf einer Höhe von weniger als 100 Teilen (auf Gewichtsbasis) pro 150000 Teile 3-Isothiazolon der vorstehenden Formel bewegt, wobei dieser Gehalt erhältlich ist durch (A) Verwendung eines Diamids der Formel

X O
| ||
$S_{1-3}$(CHCHCNHY)$_2$
|
Z

als Amidzwischenprodukt, worin X und Z Wasserstoff oder ($C_1$—$C_4$)Alkyl sind und Y die vorstehend angegebene Bedeutung besitzt, und unmittelbar vor der Zyklisierung ganz oder teilweise ein oder mehrere

Vorläufer des Nitrosamins aus dem Diamidzwischenprodukt entfernt wird (werden) durch (i) Ionenaustausch, (ii) Umkristallisation und/oder (iii) Lösungsmittelextraktion, oder (B) durch Vermeiden oder durch Verringern einer Bildung derartiger Vorläufer während der Amidierungsreaktion, durch welche das Amidzwischenprodukt gebildet wird, durch (a) (i) Herstellen des 3-Isothiazolons aus einem Zwischenprodukt, das ein Diamid enthält, das vorstehend unter (A) definiert worden ist, und (ii) Bildung des Zwischenprodukts durch Umsetzung eines geeigneten Disulfids und eines Amins in Gegenwart eines nukleophilen Spülmittels, das ein Material ist, wie ein aliphatisches oder aromatisches Mercaptan, welches im allgemeines aktiver ist als ein Amin bei einer Michael-Additionsreaktion, das jedoch nicht die Reaktanten oder das Reaktionsprodukt zersetzt, oder (b) durch Zyklisierung eines Mercaptoamids der Formel

HSC(Z)HC(X)HC(O)NHY;

worin X, Y und Z die vorstehend angegebenen Bedeutungen besitzen, mit einem Halogenierungsmittel.

7. Zubereitung nach Anspruch 6, enthaltend eines oder beide der 3-Isothiazolone der angegebenen Formel, worin R Wasserstoff ist, $R^1$ Wasserstoff oder Chlor ist und Y Methyl ist.

8. Zubereitung nach Anspruch 7, wobei das 3-Isothiazolon aus einer Mischung aus 5-Chlor-2-methyl-4-isothiazolin-3-on und 2-Methyl-4-isothiazolin-3-on besteht, wobei die Mischung 60 bis 90 Gew.-% Chlorverbindung enthält.

9. Zubereitung nach einem der Ansprüche 6 bis 8, wobei der Gehalt an Nitrosamin und einem oder mehreren Vorläufern davon weniger als 50 Teile pro 150000 Teile des Isothiazolons, bezogen auf das Gewicht, beträgt.

10. Zubereitung nach Anspruch 9, wobei der Gehalt an Nitrosamin und einem oder mehreren Vorläufern davon weniger als 20 Teile pro 150000 Teilen des Isothiazolons, bezogen auf das Gewicht, beträgt.

11. Verwendung einer Zubereitung gemäß einem der Ansprüche 6 bis 10 zur Herstellung einer kosmetischen Zubereitung (einschließlich von Toilettenartikeln) oder einer pharmazeutischen Zubereitung in einer biostatischen oder bioziden Menge.

**Revendications**

1. Un procédé de préparation d'une composition de 3-isothiazolone contenant (a) une 3-isothiazolone de formule:

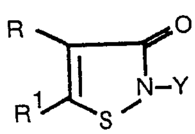

(I)

dans laquelle R et $R^1$ sont semblables ou différents et représentent un hydrogène, un halogène ou un alcoyle ($C_1$—$C_4$) et Y est un alcoyle ($C_1$—$C_8$), un cycloalcoyle ($C_3$—$C_6$), un aralcoyle ayant jusqu'à 8 atomes de carbone ou un phényle facultativement substitué à par un ou plusieurs halogènes, cyano, nitro, alcoyles ($C_1$—$C_4$), alcoxy ($C_1$—$C_4$), alcoylacylamino ($C_1$—$C_4$), carbalcoxy ($C_1$—$C_4$) ou sulfamyles, (b) un milieu pour l'isothiazolone, lequel milieu comprend de l'eau, un solvant organique polaire ou un de leurs mélanges, et (c) une quantité appropriée d'un sel de type nitrate comme stabilisant de l'isothiazolone, où l'isothiazolone est préparée par cyclisation, avec un agent d'halogénation, d'un produit intermédiaire contenant un amide, caractérisé en ce qu'une teneur en impuretés constituées de sous-produits de type nitrosamine et d'un ou plusieurs de leurs précurseurs dans le produit final stabilisé inférieure à 100 parties (en poids) pour 150 000 parties de la 3-isothiazolone est obtenue (A) par emploi comme amide intermédiaire d'un diamide de formule:

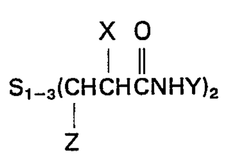

dans laquelle X et Z sont un hydrogène ou un alcoyle ($C_1$—$C_4$) et Y est comme défini ci-dessus, et immédiatement avant la cyclisation, l'élimination totale ou partielle du ou des précurseurs de la nitrosamine du diamide intermédiaire par (i) échange d'ions, (ii) recristallisation et/ou (iii) extraction par solvant ou (B) par empêchement ou réduction de la formation de ce ou ces précurseurs pendant la réaction d'amidation qui forme l'amide intermédiaire par: (a) (i) préparation de la 3-isothiazolone à partir d'un produit intermédiaire contenant un diamide comme défini en (A) ci-dessus et (ii) formation du produit intermédiaire par réaction d'un disulfure approprié et d'une amine en présence d'un épurateur nucléophile, qui est une matière telle qu'un mercaptan aliphatique ou aromatique qui est généralement plus actif qu'une amine dans une réaction d'addition de Michael, mais qui ne dégrade pas les composés réagissants

ni le produit de la réaction; ou par (b) cyclisation, avec un agent d'halogénation, d'un mercaptoamide de formule HSC(Z)HC(X)HC(O)NHY dans laquelle X, Y et Z sont comme défini ci-dessus.

2. Un procédé selon la revendication 1 dans lequel l'impureté de type nitrosamine est constituée d'une nitrosamine de formule générale:

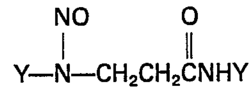

et/ou les précurseurs comprennent au moins un composé de formule

$Y—NHCH_2CH_2CONHY$ ou 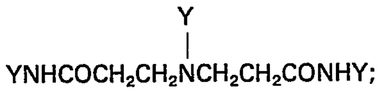;

où Y est comme défini dans la revendication 1.

3. Un procédé selon les revendications 1 et 2 où la 3-isothiazolone comprend un mélange de 5-chloro-2-méthyl-4-isothiazolone et de 2-méthyl-4-isothiazoline-3-one et Y dans les formules de la revendication 2 et $—CH_3$.

4. Un procédé selon la revendication 1 (B) (a) ou 1 (B) (b) où (a) un échange d'ions, (b) une recristallisation et/ou (c) une extraction par solvant est/sont également utilisé(e)(s) pour effectuer une élimination partielle ou complète du ou des précurseurs de nitrosamine avant la cyclisation.

5. Un procédé selon l'une quelconque des revendications précédentes dans lequel la teneur en nitrosamine et en son ou ses précurseurs est inférieure à 50 parties, de préférence inférieure à 20 parties, pour 150 000 parties de la 3-isothiazolone (en poids).

6. Une composition biocide ou biostatique contenant (1) une 3-isothiazolone de formule:

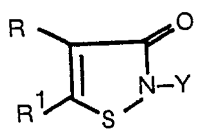

(I)

dans laquelle R et $R^1$ sont semblables ou différents et représentent un hydrogène, un halogène ou un alcoyle ($C_1—C_4$) et Y est un alcoyle ($C_1—C_8$), un cycloalcoyle ($C_3—C_6$), un aralcoyle ayant jusqu'a 8 atomes de carbone ou un phényle facultativement substitué par un ou plusieurs halogènes, cyano, nitro, alcoyles ($C_1—C_4$), alcoxy ($C_1—C_4$), alcoylacylamino ($C_1—C_4$), carbalcoxy ($C_1—C_4$) ou sulfamyles, (2) un milieu pour l'isothiazolone, lequel milieu comprend de l'eau ou un solvant organique polaire ou un mélange de ceux-ci, (3) une quantité stabilisant le cycle d'un sel de type nitrate qui stabilise la 3-isothiazolone contre le clivage du cycle, où la 3-isothiazolone a été préparée par cyclisation, avec un agent d'halogénation d'un amide intermédiaire, caractérisée en ce que la teneur en nitrosamines de formule

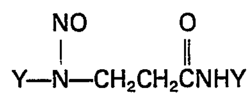

et du ou des précurseurs de celles-ci dans la composition est à un taux inférieur à 100 parties (en poids) pour 150 000 parties de 3-isothiazolone de formule ci-dessus, ledit taux pouvant être obtenu par (A) emploi comme amide intermédiaire d'un diamide de formule:

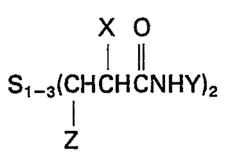

dans laquelle X et Z sont un hydrogène ou un alcoyle ($C_1—C_4$) et y est comme défini ci-dessus et, immédiatement avant la cyclisation, l'élimination totale ou partielle du ou des précurseurs de la nitrosamine du diamide intermédiaire par (i) échange d'ions, (ii) recristallisation et/ou (iii) extraction par solvant ou (B) par empêchement ou réduction de la formation de ce ou ces précurseurs pendant la réaction d'amidation qui forme l'amide intermédiaire par: (a) (i) préparation de la 3-isothiazolone à partir d'un produit intermédiaire contenant un diamide comme défini en (A) ci-dessus et (ii) formation du produit intermédiaire par réaction d'un disulfure appropriée et d'une amine en présence d'un épurateur nucléophile, qui est une matière telle qu'un mercaptan aliphatique ou aromatique qui est généralement plus actif qu'une amine dans une réaction d'addition de Michael, mais qui ne dégrade pas les composés réagissants ni le produit de la réaction; ou par (b) cyclisation, avec un agent d'halogénation, d'un

mercaptoamide de formule HSC(Z)HC(X)HC(O)NHY dans laquelle X, Y et Z sont comme définis ci-dessus.

7. Une composition selon la revendication 6 contenant l'une ou les deux 3-isothiazolones de formule indiquée dans laquelle R est un hydrogène, $R^1$ est un hydrogène ou un chlore et Y est un méthyle.

8. Une composition selon la revendication 7 dans laquelle la 3-isothiazolone comprend un mélange de 5-chloro-2-méthyl-4-isothiazoline-3-one et de 2-méthyl-4-isothiazoline-3-one, le mélange contenant 60 à 90% en poids du composé chloro.

9. Une composition selon l'une quelconque des revendications 6 à 8 dans laquelle la teneur en nitrosamine et en son ou ses précurseurs est inférieure à 50 parties pour 150 000 parties de la 3-isothiazolone, en poids.

10. Une composition selon la revendication 9 dans laquelle la teneur en nitrosamine et en son ou ses précurseurs est inférieure à 20 parties pour 150 000 parties de la 3-isothiazolone, en poids.

11. L'emploi d'une composition selon l'une quelconque des revendications 6 à 10 dans la production d'une préparation cosmétique (y compris pour la toilette) ou pharmaceutique en une quantité biostatique ou biocide.